(19) Europäisches Patentamt / European Patent Office / Office européen des brevets

(11) **EP 1 694 601 B1**

(12) **FASCICULE DE BREVET EUROPEEN**

(45) Date de publication et mention
de la délivrance du brevet:
**01.08.2018 Bulletin 2018/31**

(21) Numéro de dépôt: **04816446.1**

(22) Date de dépôt: **20.12.2004**

(51) Int Cl.:
*C01B 33/193* (2006.01)     *A61K 8/25* (2006.01)
*A61Q 11/00* (2006.01)     *C08K 3/36* (2006.01)
*C08L 83/04* (2006.01)     *H01M 2/16* (2006.01)

(86) Numéro de dépôt international:
**PCT/FR2004/003313**

(87) Numéro de publication internationale:
**WO 2005/061384 (07.07.2005 Gazette 2005/27)**

(54) **SILICE DE PRÉCIPITATION DE HAUTE STRUCTURE A FAIBLE REPRISE EN EAU, PROCÉDÉ DE PRÉPARATION ET UTILISATIONS**

HOCHSTRUKTURIERES FÄLLUNGSKIESELSÄURE MIT GERINGER WASSERAUFNAHME, HERSTELLUNGSVERFAHREN DAFÜR UND VERWENDUNGEN DAVON

HIGHLY-STRUCTURED PRECIPITATED SILICA HAVING A LOW WATER UPTAKE, PREPARATION METHOD THEREOF AND USES OF SAME

(84) Etats contractants désignés:
**AT BE BG CH CY CZ DE DK EE ES FI FR GB GR HU IE IS IT LI LT LU MC NL PL PT RO SE SI SK TR**

(30) Priorité: **19.12.2003 FR 0315063**
**08.04.2004 FR 0403700**

(43) Date de publication de la demande:
**30.08.2006 Bulletin 2006/35**

(73) Titulaire: **Rhodia Chimie SA**
**92512 Boulogne-Billancourt Cedex (FR)**

(72) Inventeurs:
• **DROMARD, Adrien**
**F-69006 Lyon (FR)**

• **CHEVALLIER, Yvonick**
**F-69270 Saint Romain-au-Mont d'Or (FR)**
• **VALERO, Rémi**
**F-01390 Saint Jean de Thurigneux (FR)**
• **PETIT, Dominique**
**F-69450 Saint Cyr-au-Mont D'or (FR)**

(74) Mandataire: **Ferri, Isabella et al**
**Solvay S.A.**
**Viale Lombardia, 20**
**20021 Bollate (MI) (IT)**

(56) Documents cités:
**EP-A- 0 407 262     WO-A-95/09127**
**WO-A-95/09128     WO-A-03/055801**

**Description**

La présente invention est relative à une nouvelle silice de précipitation de haute structure, à faible reprise en eau et à un procédé de préparation de ladite silice.

Elle est également relative à son utilisation comme charge renforçante dans les matrices à base d'élastomères, en particulier clairs ou semi-clairs, pour semelles de chaussures, dans les matrices silicones, par exemple destinées à l'enrobage des câbles électriques.

Elle concerne également son utilisation notamment comme charge et/ou support et/ou excipient dans des compositions diverses, comme des compositions alimentaires, cosmétiques, pharmaceutiques, des compositions pour la fabrication de peintures ou de papiers, des compositions destinées à la fabrication de membranes poreuses séparatrices pour batteries (« battery separators »), comme agent épaississant dans les formulations dentifrices.

Les silices dites « de précipitation » présentent souvent une forte affinité pour l'eau, en raison notamment de la présence à leur surface de groupements Si-OH avides d'eau. Les silices de précipitation les plus usuelles présentent généralement des reprises en eau (selon le test défini ci-après) supérieures à 7 %, le plus souvent de l'ordre de 8 à 10 %.

Un procédé pour préparer une silice de précipitation à faible reprise en eau (de l'ordre 4 à 6 %) fait l'objet de la demande WO 03/055801 ; la silice alors obtenue présente généralement une surface spécifique CTAB (surface externe) de 100 à 200 m$^2$/g et une prise d'huile DOP de 150 à 300 ml/100g ; il est indiqué que cette silice peut être utilisée pour le renforcement de matrices élastomères à base de silicones, notamment des matrices silicones vulcanisables à froid ou à chaud, de matrices élastomères transparentes ou translucides pour semelles de chaussures ; il est aussi mentionné que cette silice est également utilisable comme agent épaississant au sein de milieux organiques ou aqueux, notamment des pâtes dentifrices.

Des silices de précipitation de haute structure présentant une prise d'huile DOP supérieure à 250 ml/g, notamment de l'ordre de 300 à 320 ml/100g, et une surface spécifique CTAB (surface externe) de 70 à 250 m$^2$/g ont déjà été proposées comme agent épaississant ou texturant dans les compositions dentifrices (demande WO 01/93803) ; de telles silices présentent une reprise en eau supérieure à 7 %, c'est-à-dire classique des silices de précipitation.

Le Demandeur a maintenant trouvé une nouvelle silice précipitée présentant de bonnes performances de dispersion en formulation, présentant avantageusement une dispersibilité élevée au sein de matrices ou milieux solides, en particulier élastomères (clairs, semi-clairs, silicones) ou pâteux divers, voire une transmission élevée de la lumière. Ceci se traduit notamment par un bon pouvoir renforçant et/ou épaississant. Cette silice est particulièrement apte à être employée, notamment, comme charge renforçante dans les matrices à base d'élastomères, par exemple clairs ou semi-clairs, pour semelles de chaussures, dans les matrices silicones, par exemple vulcanisables à froid ou à chaud. Une application particulièrement intéressante de cette silice consiste, entre autres, dans son utlisation comme agent épaississant dans les formulations dentifrices.

Un premier objet de l'invention consiste en une silice de précipitation présentant :

- une surface spécifique CTAB de 140 à 230 m$^2$/g, de préférence de 145 à 195 m$^2$/g, plus préférentiellement de 145 à 185 m$^2$/g, tout particulièrement de 150 à 185 m$^2$/g, notamment de 150 à 180 m$^2$/g, par exemple de 155 à 175 m$^2$/g ou de 160 à 180 m$^2$/g
- une prise d'huile DOP supérieure à 300 ml/g, de préférence supérieure à 310 ml/100g, plus préférentiellement de 315 à 450 ml/100g, tout particulièrement de 320 à 400 ml/100g, notamment de 340 à 380 ml/100g
- une reprise en eau inférieure à 6 % en masse et de préférence supérieure à 3 % en masse, tout particulièrement supérieure ou égale à 4 % en masse et inférieure ou égale à 5,8 % en masse
- un pH de 3,5 à 7,5, de préférence de 4 à 7, tout particulièrement de 4 à 6
- un taux d'anion résiduel, exprimé en sulfate de sodium, inférieur ou égal à 2 % en masse, de préférence inférieur ou égal à 1,5 % en masse, particulièrement inférieur ou égal à 1 % en masse, et tout particulièrement inférieur ou égal à 0,5 % en masse
- une taille moyenne ou un diamètre médian de particules inférieur à 30 μm ou compris entre 30 μm et 20 mm.

Selon une première variante de l'invention, la silice présente une taille moyenne ou un diamètre médian de particules inférieur à 30 μm, de préférence inférieur à 20 μm, en particulier compris entre 5 et 15 μm, notamment entre 8 et 13 μm.

Selon une seconde variante de l'invention, la silice présente une taille moyenne ou un diamètre médian de particules compris entre 30 μm et 20 mm.

La silice selon cette seconde variante de l'invention présente, de manière très préférée, une surface spécifique CTAB de 145 à 185 m$^2$/g, notamment de 150 à 180 m$^2$/g, tout particulièrement de 155 à 175 m$^2$/g.

La surface spécifique CTAB est la surface externe déterminée selon la norme NFT 45-007 (novembre 1987).

La prise d'huile DOP est déterminée selon la norme ISO 787/5 en mettant en oeuvre du dioctylphtalate.

L'affinité d'une silice vis-à-vis de l'eau traduite par sa caractéristique dite de "reprise en eau" reflète la tendance plus ou moins marquée que présentent les molécules d'eau à s'adsorber sur la surface de la silice.

Le principe du test de mesure de cette caractéristique consiste à placer l'échantillon de silice préalablement séché, dans des conditions d'humidité relatives données et pendant une durée prédéfinie ; la silice s'hydrate, ce qui fait passer la masse de l'échantillon d'une valeur initiale m (à l'état séché) jusqu'à une valeur finale (m+dm). On désignera spécifiquement par "reprise en eau" d'une silice le rapport dm/m exprimé en pourcentage calculé pour un échantillon de silice soumis aux conditions suivantes lors du test :

- séchage préliminaire : 8 heures, à 105 °C ;
- hydratation : 24 heures, à 20 °C, et sous une humidité relative de 70 %.

Le protocole expérimental mis en oeuvre consiste à :

- peser exactement environ 2 g de la silice à tester ;
- sécher pendant 8 heures la silice ainsi pesée dans une étuve réglée à une température de 105 °C ;
- déterminer la masse m de la silice séchée obtenue à l'issue de ce séchage ;
- disposer pendant 24 heures, à 20 °C, la silice séchée obtenue dans un récipient fermé (par exemple dans un dessicateur) contenant un mélange eau/glycérine avec un rapport massique eau/glycérine de 35/65, de façon à ce que l'humidité relative du milieu fermé soit de 70 % ;
- déterminer la masse (m+dm) de la silice obtenue suite à ce traitement de 24 heures à 70 % d'humidité relative, la mesure de cette masse étant effectuée immédiatement après avoir sorti la silice du dessicateur, de façon à éviter une variation de la masse de la silice sous l'influence du changement d'hygrométrie entre le milieu à 70 % d'humidité relative et l'atmosphère du laboratoire.

Le pH de la silice est mesuré selon la norme ISO 787/9 (pH d'une suspension à 5 % en poids de silice dans l'eau désionisée).

La silice selon l'invention peut se présenter sous forme de billes, de granulés (ou autres agrégats), ou, de manière préférée, de poudre, possédant une taille moyenne et un diamètre médian de particules d'au plus 20 mm.

La silice selon la première variante de l'invention peut se présenter sous forme de billes, de granulés (ou autres agrégats), ou, de manière préférée, de poudre, possédant une taille moyenne et un diamètre médian de particules inférieurs à 30 $\mu$m, de préférence inférieurs à 20 $\mu$m, en particulier compris entre 5 et 15 $\mu$m, notamment entre 8 et 13 $\mu$m. Cette silice est particulièrement adaptée pour être employée, à titre de charge renforçante dans des matrices à base d'élastomères, notamment clair(s) ou semi-clair(s), pour semelles de chaussures, à titre de charge renforçante dans des matrices à base de silicone(s).

La silice selon la seconde variante de l'invention peut se présenter sous forme de billes, de granulés (ou autres agrégats), ou, de manière préférée, de poudre, possédant une taille moyenne et un diamètre médian de particules compris entre 30 $\mu$m et 20 mm. Il peut notamment s'agir de poudre présentant un diamètre médian de particules d'au moins 30 $\mu$m, de préférence d'au moins 50 $\mu$m, et inférieur à 350 $\mu$m, de préférence inférieur à 180 $\mu$m ; cette silice est particulièrement apte à être utilisée comme agent épaississant ou texturant au sein de compositions dentifrices, comme charge renforçante dans des matrices à base de silicone(s).

Il peut également s'agir de granulés (ou autres agrégats) présentant une taille moyenne de particules compris entre 2 et 20 mm.

La taille moyenne des particules de silice peut être déterminée selon la norme NF X 11507 (décembre 1970) par tamisage à sec et détermination du diamètre correspondant à un refus cumulé à 50 %.

Le diamètre médian des particules de silice peut être déterminé par diffraction laser selon la norme NF X 11-666. Le granulomètre utilisé est du type MALVERN MASTERSIZER.

Critères de mesure

* concentration optique : 12 $\pm$ 2 %
* liquide de mesure : eau déminéralisée dégazée
* absence d'ultrasons
* absence de dispersant
* durée de la mesure : 10 secondes

La silice de précipitation selon l'invention, présente généralement un diamètre médian d50 des particules, après désagglomération aux ultra-sons, d'au plus 35 $\mu$m, de préférence d'au plus 30 $\mu$m, tout particulièrement d'au plus

25 μm, notamment d'au plus 15 μm, par exemple d'au plus 10 μm.

Le diamètre médian d50 de la silice après désagglomération par ultra-sons est mesuré au granulomètre MALVERN MASTERSIZER, selon le test suivant :

La puissance des ultra-sons dans le granulomètre MALVERN MASTERSIZER étant réglée sur la graduation maximale de 20, on introduit une quantité de silice de façon à obtenir une concentration optique de 12 ± 2 %.

On mesure le diamètre médian d50 et le pourcentage de particules de silice de diamètre supérieur à 51 μm après avoir maintenu les ultra-sons dans la cuve pendant 60 secondes, la cuve étant homogénéisée par circulation de la suspension au moyen d'une pompe centrifuge. La mesure est enregistrée 10 secondes après l'arrêt des ultra-sons.

L'aptitude de la silice selon l'invention à se disperser ou à se désagglomérer peut également être appréciée par une mesure granulométrique (par diffraction laser), effectuée sur une suspension de silice préalablement désagglomérée par ultra-sonification (rupture des objets de 0,1 à quelques dizaines de microns). La désagglomération sous ultra-sons est effectuée à l'aide d'un sonificateur VIBRACELL BIOBLOCK (600 W), équipé d'une sonde de diamètre 19 mm. La mesure granulométrique est effectuée par diffraction laser sur un granulomère SYMPATEC.

On pèse dans un pilulier (hauteur : 6 cm et diamètre : 4 cm) 2 grammes de silice et l'on complète à 50 grammes par ajout d'eau permutée : on réalise ainsi une suspension aqueuse à 4 % de silice qui est homogénéisée pendant 2 minutes par agitation magnétique. On procède ensuite à la désagglomération sous ultra-sons comme suit : la sonde étant immergée sur une longueur de 4 cm, on règle la puissance de sortie de manière à obtenir une déviation de l'aiguille du cadran de puissance indiquant 20 %. La désagglomération est effectuée pendant 420 secondes. On réalise ensuite la mesure granulométrique en introduisant dans la cuve du granulomère un volume V (exprimé en ml) de la suspension homogénéisée nécessaire pour obtenir une densité optique de l'ordre de 20.

Un facteur de désagglomération $F_D$ est alors donné par l'équation :

$$F_D = 10 \times V/\text{densité optique de la suspension mesurée par le granulomètre (cette densité optique est de l'ordre de 20).}$$

Ce facteur de désagglomération $F_D$ est indicatif du taux de particules de taille inférieure à 0,1 μm qui ne sont pas détectées par le granulomètre. Ce facteur est d'autant plus élevé que la silice présente une aptitude à la désagglomération élevée.

La valeur du diamètre médian $\varnothing_{50}$ que l'on obtient selon ce test est d'autant plus faible que la silice présente une aptitude à la désagglomération élevée.

De préférence, la silice selon l'invention possède un diamètre médian $\varnothing_{50}$, après désagglomération aux ultra-sons, inférieur à 6 μm, en particulier inférieur à 5 μm, par exemple inférieur à 3,5 μm.

La silice selon l'invention présente, en général, un facteur de désagglomération aux ultra-sons $F_D$ supérieur à 5,5 ml, en particulier supérieur à 7,5 ml, par exemple supérieur à 11,5 ml.

Les silices selon l'invention présentent de préférence une surface spécifique BET telle que la différence BET-CTAB soit d'au plus 30 m$^2$/g, de préférence d'au plus 25 m$^2$/g, plus préférentiellement d'au plus 20 m$^2$/g, en particulier d'au plus 10 m$^2$/g.

La surface spécifique BET est déterminée selon la méthode de BRUNAUER - EMET - TELLER décrite dans "The journal of the American Chemical Society", Vol. 60, page 309, février 1938 et correspondant à la norme NFT 45007 (novembre 1987).

Par ailleurs, les silices selon l'invention, présentent généralement une densité de remplissage à l'état tassé, mesurée selon la norme ISO 787/11, d'au plus 0,3 g/ml, de préférence de 0,04 à 0,3 g/ml, plus préférentiellement de 0,05 à 0,3 g/ml, notamment de 0,05 à 0,2 g/ml ; cette densité de remplissage à l'état tassé peut être également comprise entre 0,1 à 0,3 g/ml, notamment entre 0,1 et 0,27 g/ml, en particulier entre 0,15 et 0,25 g/ml.

La perte au feu (PAF) de la silice de l'invention, mesurée selon la norme ISO 3262/11, après traitement à 1000 °C, est généralement telle que la différence PAF - humidité soit inférieure à 3,2 %, de préférence inférieure à 3 %, tout particulièrement inférieure à 2,7 %.

L'humidité est la teneur en eau résiduelle mesurée selon la norme ISO 787/2, après traitement thermique à 105 °C pendant 2 heures.

L'humidité de la silice selon l'invention, notamment lorsqu'elle est destinée à être utilisée comme charge dans des matrices silicones, est généralement inférieure à 5 %, de préférence inférieure à 4 %, par exemple d'au plus 3 %, de la masse totale de l'échantillon.

La silice selon l'invention peut présenter en outre un taux de transmission d'au moins 70 % à un indice de réfraction dans le glycérol situé entre 1,450 et 1,467.

L'indice de réfraction considéré est celui correspondant à la suspension la plus transparente (transmission maximum) de cette silice dans diverses solutions eau-glycérol, transparence déterminée par la transmission à 589 nm avec un spectrophotomètre. Chaque suspension est obtenue par dispersion de 2 g de silice dans 18 g de solution eau-glycérol, puis désaération sous léger vide avant lecture de la transmission (lecture faite avec, comme produit de référence, la solution eau-glycérol sans silice) sur le spectrophotomètre et l'indice de réfraction sur un réfractomètre.

Un deuxième objet de l'invention consiste en un procédé de préparation de la silice de haute structure et faible reprise en eau décrite ci-dessus, comprenant les étapes successives suivantes :

- (a) réaliser un pied de cuve initial de température comprise entre 80 et 100 °C, de préférence supérieure ou égale à 90 °C, comprenant de l'eau et un silicate, la concentration en silicate dans ledit pied de cuve, exprimée en équivalent $SiO_2$, étant inférieure ou égale à 15 g/L ;

- (b) addition, à une température comprise entre 80 et 100 °C, de préférence 90 et 100 °C, d'un agent acidifiant, amener le pH du milieu jusqu'à une valeur comprise entre 7 et 8, de préférence jusqu'à une valeur comprise entre 7,2 et 7,8, et avantageusement entre 7,3 et 7,7 (typiquement jusqu'à une valeur sensiblement égale à 7,5) ;

- (c) dans le milieu ainsi réalisé, effectuer, à une température comprise entre 80 et 100 °C, de préférence entre 90 et 100 °C, l'addition simultanée d'un silicate et d'un agent acidifiant, les quantités respectives de silicate et d'agent acidifiant ajoutées au cours du temps étant spécifiquement choisies de façon à ce que, pendant toute la durée de l'addition :

    - le pH du milieu réactionnel reste compris entre 7 et 8, et avantageusement entre 7,2 et 7,8 ; et

    - la concentration en silicium dans le milieu, exprimée en équivalent $SiO_2$, reste inférieure ou égale à 35 g/L ;

- (d) ajouter, à une température comprise entre 80 et 100 °C, de préférence entre 90 et 100 °C, un agent acidifiant au milieu obtenu à l'issue de l'étape (c) de façon à porter le milieu à un pH compris entre 3 et 6,5 ;

- (e) filtrer la dispersion aqueuse de silice obtenue ;

- (f) sécher le gâteau de filtration réalisé à l'issue de la filtration, de préférence en le lavant, au préalable ;

- (g) éventuellement, broyer ou microniser la silice obtenue à l'issue de l'étape (f),

ledit procédé étant caractérisé en ce que le gâteau de filtration présente, préalablement à son séchage à l'étape (f), une perte au feu à 1000 °C supérieure à 82 % en masse de préférence d'au moins 84 % en masse tout particulièrement de 84 à 88 % en masse. Les silicates mis en oeuvre dans les étapes (a) et (c) du procédé peuvent être choisis parmi toutes les formes courantes de silicates. Avantageusement, les silicates utilisés selon l'invention sont des silicates alcalins, comme par exemple les silicates de sodium ou de potassium.

De façon particulièrement préférée, le silicate de l'étape (a) est un silicate de sodium, de même que celui ajouté lors de l'étape (c). Le silicate de sodium mis en oeuvre est alors généralement caractérisé par un rapport pondéral $SiO_2/Na_2O$ compris entre 2 et 4, avantageusement entre 3 et 3,6, ce rapport pondéral $SiO_2/Na_2O$ étant de préférence compris entre 3,3 et 3,5 (typiquement ce rapport est sensiblement égal à 3,4).

Le pied de cuve de l'étape (a) du procédé se présente le plus souvent sous la forme d'une solution aqueuse de silicate dont la concentration est, de façon caractéristique, inférieure ou égale à 15 g/L. Typiquement, la concentration en silicate dans le pied de cuve de l'étape (a), exprimée en équivalent $SiO_2$, est comprise entre 1 et 15 g/L. Cette concentration en silicate dans le pied de cuve de l'étape (a), exprimée en équivalent $SiO_2$ est avantageusement inférieure ou égale à 10 g/L, et de préférence inférieure ou égale à 9 g/L.

Le pied de cuve de l'étape (a) possède généralement un pH de l'ordre de 9 à 13.

L'étape (b) du procédé de l'invention consiste spécifiquement à faire diminuer cette valeur du pH, par addition d'un agent acidifiant, de façon à amener le pH du milieu dans la plage de 7 à 8, où il a été mis en évidence que la réaction de précipitation de la silice s'effectue de façon optimale. Par "agent acidifiant", on entend tout composé acide minéral ou organique susceptible de pouvoir mener à une diminution du pH du pied de cuve. Ainsi, à titre d'agent acidifiant, on peut avantageusement mettre en oeuvre un acide minéral tel que l'acide sulfurique, l'acide chlorhydrique ou l'acide nitrique, ou bien encore un acide organique tel que l'acide acétique, l'acide formique ou l'acide carbonique.

De manière avantageuse, aucun électrolyte n'est ajouté au cours du procédé, en particulier dans l'étape (a). Le terme d'électrolyte s'entend ici dans son acceptation normale, c'est-à-dire qu'il désigne toute substance ionique ou moléculaire qui, lorsqu'elle est en solution, se décompose ou se dissocie pour former des ions ou des particules chargées (les électrolytes usuels sont des sels des métaux alcalins et alcalino-terreux, notamment le sel du métal de silicate de départ et de l'agent acidifiant, comme le chlorure de sodium dans le cas de la réaction d'un silicate de sodium avec l'acide chlorhydrique ou le sulfate de sodium dans le cas de la réaction d'un silicate de sodium avec l'acide sulfurique).

L'agent acidifiant mis en oeuvre dans l'étape (b) du procédé est de préférence l'acide sulfurique, en particulier lorsque le silicate présent dans le pied de cuve initial est un silicate alcalin. De façon générale, l'agent acidifiant de l'étape (b) est le plus souvent introduit sous la forme d'une solution aqueuse, de préférence diluée, généralement de normalité comprise entre 0,25 et 8 N. Ainsi, dans l'étape (b), la diminution du pH du milieu peut avantageusement être réalisée par addition d'une solution aqueuse d'acide sulfurique de concentration comprise entre 10 et 350 g/L, et de préférence entre 50 et 250 g/L.

Quelle que soit la nature exacte de l'agent acidifiant de l'étape (b), cet agent acidifiant doit être mis en oeuvre de façon telle que son addition conduise à une diminution du pH du milieu jusqu'à une valeur comprise entre 7 et 8. La quantité d'agent acidifiant à mettre en oeuvre dans ce cadre est généralement déterminée en pratique en mesurant l'évolution du pH au cours de l'addition, l'addition de l'agent acidifiant de l'étape (b) étant poursuivie jusqu'à ce que le pH atteigne la valeur recherchée.

Par ailleurs, l'addition de l'étape (b) s'effectue de préférence de façon progressive, c'est à dire avantageusement, en règle générale, avec une durée d'addition comprise entre 3 et 60 minutes, le plus souvent au moins égale à 5 minutes, et de préférence au moins égale à 10 minutes. Cette durée d'addition est toutefois avantageusement inférieure à 30 minutes.

Selon un mode particulier de réalisation envisageable pour l'étape (b), cette étape peut inclure un processus de mûrissement, qui, le cas échéant, est réalisé en laissant le milieu évoluer pendant une durée généralement comprise entre 5 et 30 minutes, de préférence à une température comprise entre 90 et 100 °C, étant entendu que, suite à ce mûrissement, le pH milieu réactionnel est adapté si nécessaire, notamment par ajout d'un agent acidifiant, de sorte qu'à l'issue de l'étape (b) le pH du milieu se situe dans la gamme de pH comprise entre 7 et 8, et avantageusement dans les gammes préférentielles précitées.

Suite à l'étape (b), par laquelle le pH du milieu réactionnel est amené dans la zone préférentielle de 7 à 8, et de préférence aux environs de 7,5, l'étape (c) du procédé consiste à poursuivre le processus de précipitation de silice, en introduisant du silicate additionnel, et en maintenant spécifiquement le pH du milieu dans la zone comprise entre 7 et 8, de préférence à une valeur sensiblement constante, cette valeur constante étant alors de préférence proche de 7,5, c'est-à-dire généralement comprise entre 7,3 et 7,7.

Pour ce faire, le silicate de l'étape (c) est introduit conjointement à un agent acidifiant, qui s'oppose à l'augmentation de pH qui serait observé en ajoutant le silicate seul. De préférence, l'étape (c) du procédé de l'invention est conduite immédiatement après obtention pour le milieu du pH recherché dans l'étape (b).

L' "addition simultanée" du silicate et de l'agent acidifiant qui est réalisée lors de l'étape (c) consiste avantageusement en une addition continue de silicate dans le milieu au cours de laquelle on mesure le pH du milieu, et pendant laquelle on régule la valeur de ce pH par introduction de l'agent acidifiant, cette introduction de l'agent acidifiant pouvant par exemple être réalisée dès que le pH du milieu devient supérieur à une valeur de contrôle comprise entre 7 et 8, cette valeur de contrôle étant généralement fixée aux alentours de 7,5. Par ce moyen, on arrive à maintenir dans le milieu une valeur sensiblement constante du pH, c'est-à-dire variant avantageusement à +/- 0,2 unité de pH (de préférence à +/- 0,1 unité de pH) autour d'une valeur fixe, généralement comprise entre 7,3 et 7,7.

Alternativement, l'addition simultanée de l'étape (c) peut également consister en une addition continue d'agent acidifiant dans le milieu, le pH étant alors régulé au cours de l'addition par introduction du silicate cette introduction du silicate pouvant là encore être par exemple réalisée dès que le pH du milieu devient inférieur à une valeur de contrôle comprise entre 7 et 8, fixée le plus souvent aux alentours de 7,5. Par ce moyen, on arrive également à maintenir le milieu à un pH sensiblement constant, c'est-à-dire variant avantageusement à +/- 0,2 unité de pH (de préférence à +/- 0,1 unité de pH) autour d'une valeur fixe, généralement comprise entre 7,3 et 7,7.

Selon encore un autre mode de mise en oeuvre envisageable, l'addition simultanée de l'étape (c) peut également consister en une addition continue à la fois d'agent acidifiant et de silicate, avec des concentrations et des débits calculés de façon à ce que, durant toute la durée de l'addition, le pH du milieu reste compris entre 7 et 8, et de préférence entre 7,2 et 7,8. Dans ce cas, le pH du milieu a généralement tendance à évoluer au cours de l'étape (c), tout en restant dans la gamme précitée, mais il peut, dans certains cas, demeurer sensiblement égal à une valeur constante, avantageusement de l'ordre de 7,5. Dans ce cadre, on préfère généralement que, tout au long de l'étape (c), les débits instantanés correspondant à la quantité de fonctions silicates (exprimées en équivalent molaire de NaOH) introduites par seconde (notée $d_S$), et la quantité de fonctions acides (en moles) introduites par seconde (notée $d_A$), soient tels que le rapport $d_S/d_A$ reste en permanence compris entre 1,01 et 1,09, et de préférence entre 1,02 et 1,07.

Quel que soit le mode exact de mise en oeuvre de l'étape (c), le silicate et l'agent acidifiant utilisés sont le plus souvent identiques à ceux mis en oeuvre dans les étapes (a) et (b). Ainsi, le silicate de l'étape (c) est de préférence un silicate alcalin, avantageusement un silicate de sodium, et l'agent acidifiant est préférentiellement un acide minéral fort, le plus souvent l'acide sulfurique.

Dans la mesure où, lors de l'addition simultanée de l'étape (c), la concentration en silicium dans le milieu (exprimée en équivalent $SiO_2$) doit, de façon caractéristique, être maintenue inférieure ou égale à 35 g/L, le silicate introduit dans le milieu réactionnel lors de l'étape (c) est généralement sous la forme d'une solution aqueuse diluée, c'est à dire de concentration, exprimée en équivalent $SiO_2$, avantageusement comprise entre 10 et 360 g/L, de préférence inférieure à 300 g/L et avantageusement inférieure à 270 g/L, et ce tout particulièrement lorsqu'on utilise des silicates alcalins tels que les silicates de sodium. De la même façon, l'agent acidifiant est le plus souvent sous la forme d'une solution aqueuse diluée, qui possède généralement une normalité comprise entre 0,25 et 8 N, et de préférence entre 0,5 et 4 N. Ainsi, dans le cas spécifique de l'utilisation d'une solution aqueuse d'acide sulfurique à titre d'agent acidifiant de l'étape (c), par exemple, la concentration de la solution est avantageusement comprise entre 25 et 380 g/L, et de préférence entre 50 et 300 g/L.

Il est à souligner que, compte tenu de la mise en oeuvre de concentrations diluées dans le milieu de précipitation des silices, les concentrations en sels dans ce milieu, notamment liées à la réaction du silicate et de l'agent acidifiant, sont de façon caractéristique, extrêmement faibles, ce qui se traduit par une faible force ionique au sein du milieu de précipitation mis en oeuvre.

Sans vouloir être lié en aucune façon à une théorie particulière, il semble pouvoir être avancé que le contrôle du pH et des concentrations mis en oeuvre permet de minimiser la formation de groupements de surface SiOH.

De façon à améliorer encore le contrôle de la formation de la silice, il est particulièrement avantageux de réaliser l'addition simultanée de l'étape (c), avec des débits de silicate et d'agent acidifiant relativement faibles, c'est à dire, le plus souvent, avec une durée d'addition de l'étape (c) de préférence comprise entre 15 et 300 minutes, et de préférence entre 30 et 100 minutes. De telles durées d'addition mènent en effet généralement à l'obtention de particules de silices présentant des taux de groupements Si-OH en surface extrêmement réduits.

De façon générale, l'étape (c) du procédé de l'invention est conduite sous agitation à une température comprise entre 80 et 100 °C, et généralement à la même température que l'addition de l'étape (b). Ainsi, la température de mise en oeuvre de l'étape (c) peut avantageusement être comprise entre 90 et 100 °C, et elle est de préférence de l'ordre de 95 °C. Selon une variante particulière du procédé, et ce notamment pour la préparation de silices pouvant être utilisées dans des applications autres qu'alimentaires, dentifrices, cosmétiques ou pharmaceutiques, on peut introduire dans le milieu réactionnel, au cours de l'étape (c), de préférence à la fin de cette étape (c'est à dire typiquement durant la période correspondant au dernier quart de cette étape, généralement au cours des 5 à 15 dernières minutes de cette étape) un composé à base d'aluminium, de préférence un sel de nature acide comme un sulfate d'aluminium, ou, alternativement, un composé de nature basique tel qu'un aluminate de sodium. La quantité de composé d'aluminium introduite dans ce cadre est généralement telle qu'au sein du milieu réactionnel, le ratio $Al/SiO_2$ est compris entre 0,1 et 1 % en masse, ce ratio étant de préférence au plus égal à 0,6 %, et de préférence inférieur ou égal à 0,5 %.

Quel que soit le mode exact de réalisation de l'étape (c), à l'issue de cette étape, le milieu réactionnel est spécifiquement à un pH compris entre 7 et 8, et de préférence de l'ordre de 7,5.

En fonction des applications envisagées pour la silice, l'étape (d) d'acidification du milieu dans la zone de pH de 3 à 6,5 peut être modulée par la quantité d'agent acidifiant additionné. De préférence, le pH du milieu atteint à l'issue de l'étape (d) est compris entre 3,2 et 5,5.

L'agent acidifiant de l'étape (d) peut indifféremment être identique ou différent de celui ou ceux mis en oeuvre dans les étapes (b) et (c). De préférence, cet agent acidifiant de l'étape (d) est introduit dans le milieu sous la forme d'une solution aqueuse de normalité comprise entre 0,25 et 8 N. Avantageusement, il s'agit d'une solution aqueuse d'acide sulfurique, généralement à une concentration comprise entre 25 et 380 g/L le cas échéant.

L'ensemble des étapes (a), (b), (c) et (d) du procédé est de préférence conduit à une température comprise entre 90 et 100 °C, et avantageusement à une température comprise entre 93 et 97 °C, et encore plus avantageusement à une température sensiblement égale à 95 °C tout au long du procédé.

Selon une variante avantageuse du procédé de l'invention, les dispersions aqueuses de silice obtenues à l'issue des étapes (c) et (d) peuvent être soumises à une étape de mûrissement, généralement conduite, le cas échéant, en laissant le milieu, de préférence sous agitation, à une température comprise entre 90 et 100 °C, pendant une durée qui peut être avantageusement comprise entre 15 et 240 minutes, et de préférence pendant une durée supérieure à 30 minutes, la température lors du mûrissement étant préférentiellement sensiblement constante (le cas échéant, avantageusement sensiblement égale à 95 °C), ou bien croissante (généralement par pallier(s) le cas échéant) dans la gamme de température allant de 90 à 100 °C.

Il est à souligner que l'addition d'un composé d'aluminium, notamment de type sulfate d'aluminium, qui est envisageable à la fin de l'étape (c) peut également être conduite au cours de l'étape (d), ou bien encore au cours de l'étape de mûrissement ultérieure, lorsque cette étape est mise en oeuvre. Ainsi, de façon générale, cette addition d'un composé à base d'aluminium dans le milieu peut avoir lieu entre l'étape (c) et l'étape (e).

Les étapes (e) et (f) du procédé consistent, de façon globale, à récupérer une silice sous forme solide à partir de la dispersion obtenue à l'issue des étapes précédentes.

Le plus souvent, lors de cette étape (e), la dispersion obtenue à l'issue de l'étape (d) et de l'éventuelle étape de mûrissement ultérieure, est soumise à une filtration sur filtre presse ou à une filtration sous vide en utilisant un filtre rotatif, un filtre à bande, ou encore un filtre plan, cette filtration conduisant à l'obtention d'un "gâteau de silice". Le gâteau de silice obtenu est alors le plus souvent soumis à une étape de lavage, généralement par de l'eau, de préférence d'une durée suffisamment longue, de façon à réduire sa teneur en sels, et il est ensuite soumis à l'étape (f) à un séchage, notamment par une atomisation adaptée, et par exemple à l'aide d'un atomiseur à turbines, à buse, à pression liquide ou bifluide.

Dans ce cadre, on délite généralement le gâteau de silice au préalable, de façon à former une bouillie de silice de viscosité suffisamment faible pour assurer son pompage jusqu'à l'atomiseur.

Selon l'invention, cette bouillie présente une perte au feu à 1000 °C supérieure à 82 % en masse, de préférence d'au moins 84 % en masse, plus particulièrement de 84 à 88 % en masse.

Le cas échéant, l'opération de délitage peut par exemple être réalisée d'une manière connue en soumettant le gâteau à une action mécanique et éventuellement à une action chimique (addition d'un acide ou d'un composé à base d'aluminium).

Le plus souvent, la bouillie de faible viscosité issue d'un tel délitage se présente sous la forme d'une dispersion aqueuse de silice qui est directement pompée vers un atomiseur pour l'étape (f).

Les silices séchées obtenues à l'issue de l'étape (f) peuvent éventuellement être soumises à une étape d'agglomération, notamment par compression directe, par une granulation en voie humide (c'est-à-dire avec utilisation d'un liant tel que l'eau), par extrusion et, de préférence, par compactage à sec. Lorsque l'on met en oeuvre cette dernière technique, il peut s'avérer avantageux, avant de procéder au compactage, de désaérer (opération aussi appelée prédensification ou dégazage) les produits pulvérulents de manière à éliminer l'air inclus dans ceux-ci et assurer un compactage plus régulier. A l'issue de l'étape d'agglomération, les produits peuvent être calibrés à une taille désirée, par exemple par tamisage. La silice précipitée compactée susceptible d'être obtenue se présente alors avantageusement sous la forme de granulés. Le cas échéant, ces granulés peuvent se présenter sous des formes les plus diverses. A titre d'exemple, on peut notamment citer les formes sphérique, cylindrique, parallélépipédique, de pastille, de plaquette, de boulette, d'extrudé à section circulaire ou polylobée. Les dimensions moyennes de ces granulés sont préférentiellement comprises entre 2 et 20 mm.

La silice obtenue à l'issue de l'étape (f), puis éventuellement agglomérée, présente préférentiellement une taille moyenne ou un diamètre médian de particules compris entre 30 $\mu$m et 20 mm.

La silice obtenue à l'issue de l'étape (f), puis éventuellement agglomérée, peut être ensuite micronisée ou, de préférence, broyée.

La silice alors obtenue présente préférentiellement une taille moyenne ou un diamètre médian de particules inférieur à 30 $\mu$m, de préférence inférieur à 20 $\mu$m, en particulier compris entre 5 et 15 $\mu$m, notamment entre 8 et 13 $\mu$m

La micronisation peut être effectuée avec un microniseur, comme un broyeur à jet d'air.

Le broyage peut être mis en oeuvre en particulier à l'aide d'un broyeur mécanique, par exemple du type ACM, Forplex, notamment un broyeur sélecteur à marteaux.

Les silices de précipitation selon la présente invention présentent une très bonne aptitude à la dispersion. Elles sont particulièrement adaptées, de manière avantageuse lorsqu'elles présentent une taille moyenne ou un diamètre médian de particules inférieur à 30 $\mu$m, de préférence inférieur à 20 $\mu$m, notamment compris entre 5 et 15 $\mu$m, par exemple entre 8 et 13 $\mu$m (silices selon la première variante de l'invention), pour une utilisation

- à titre de charge renforçante dans des matrices à base d'élastomère(s), en particulier clair(s) ou semi-clair(s), pour semelles de chaussures
- à titre de charge renforçante dans des matrices à base de silicone(s), notamment des matrices élastomères silicones vulcanisables à chaud ou à froid, auxquelles elles confèrent de bonnes propriétés rhéologiques, tout en leur procurant des propriétés mécaniques très satisfaisantes

Les silices selon l'invention trouvent une application particulièrement avantageuse dans le renforcement de matrices à base d'élastomères, en particulier clairs ou semi-clairs, destinés à la fabrication de semelles de chaussures ; ces silices dispersibles permettent un renforcement élevé des matrices transparentes ou translucides utilisées pour la confection de pièces en caoutchouc transparentes ou translucides constitutives de semelles de chaussures. De manière avantageuse, elles permettent l'obtention de matrices renforcées ayant une très bonne transparence.

La quantité de silice selon l'invention pouvant être utilisée dans ce type de matrice est généralement comprise entre 10 et 50 %, notamment entre 20 et 40 %, par rapport au poids d'élastomère(s).

Les silices selon l'invention trouvent une application également avantageuse dans le renforcement de compositions (matrices) organosiliciques élastomères ou pâteuses vulcanisables à chaud (silicones HTV par exemple) ou à froid, destinées notamment à une fonction d'isolation, comme l'enrobage de câbles électriques. Lesdites matrices à base de silicone, en particulier celles destinées à une fonction d'isolation, peuvent être mises en forme par extrusion, avant d'être réticulées. La faible valeur de reprise en eau des silices de l'invention permet d'éviter ou de limiter la formation de bulles notamment lors de l'extrusion. Ces matrices à base de silicone peuvent être également mises en forme par moulage. Les silices selon l'invention confèrent avantageusement aux matrices silicones de très bonnes propriétés électriques et mécaniques, notamment au niveau de la résistance à la déchirure, de la résistance à la rupture. La nature du ou des organopolysiloxanes vulcanisables présents dans ce type de composition, ainsi que celle des agents de vulcanisation et autres additifs éventuellement présents, de même que les conditions de vulcanisation sont bien connues de l'homme de métier ; celles-ci sont notamment décrites dans la demande WO 03/055801.

La quantité de silice selon l'invention pouvant être mise en oeuvre pour le renforcement desdites matrices à base de silicones peut aller de 3 à 20 % lorsqu'il s'agit de pâtes silicones, ou de 5 à 50 %, de préférence de 10 à 40 % lorsqu'il s'agit d'une composition de nature élastomère.

Une application possible des silices selon l'invention réside dans leur utilisation comme support de liquide, notamment du fait de leur bonne capacité d'absorption et d'une coulabilité très satisfaisante.

On peut citer comme liquides, les liquides organiques tels que les acides organiques, les agents tensioactifs, par exemple de type anionique ou non-ionique, les additifs organiques pour caoutchouc / polymères, les pesticides.

A titre de liquides, on utilise de préférence ici surtout des additifs liquides tels que des arômes, des colorants, des compléments liquides d'alimentation (notamment d'alimentation animale (par exemple la vitamine E, l'acétate de vitamine E, le chlorhydrate de choline)) ou des agents conservateurs, de préférence des acides carboxyliques (acide propionique par exemple).

Les compositions conditionnées comprenant au moins un liquide absorbé sur un support formé par une silice selon l'invention, présentent préférentiellement une teneur en liquide d'au moins 50 % en poids, en particulier comprise entre 50 et 75 % en poids, par exemple entre 50 et 65 % en poids.

Par ailleurs, les silices de l'invention peuvent être mises en oeuvre comme charge et/ou support et/ou excipient dans des compositions diverses, comme des compositions alimentaires, cosmétiques, pharmaceutiques, des compositions pour la fabrication de peintures ou de papiers.

On peut également mentionner l'utilisation, par exemple en quantité de l'ordre de 60 % en poids, de la silice de l'invention, à titre de support de solvant et/ou d'huile, dans des compositions à base de polymères destinées à la préparation de membranes poreuses séparatrices de batteries (« battery separator ») ; le solvant et/ou l'huile supportés, une fois extraits après extrusion/calandrage, donnent naissance à un réseau de pores.

La silice selon l'invention, de manière avantageuse lorsqu'elle présente une taille moyenne ou un diamètre médian de particules compris entre 30 $\mu$m et 20 mm (silice selon la seconde variante de l'invention), peut être incorporée au sein de compositions dentifrices, lors de la préparation desdites compositions, qui peuvent se présenter sous forme de pâte ou de gel, et peut permettre ainsi d'épaissir ces compositions ou de leur apporter de la texture.

Selon l'invention, ladite silice peut être utilisée comme agent épaississant ou texturant à raison de 0,1 à 20 %, de préférence de 0,5 à 15 %, tout particulièrement de 1 à 10 %, en poids de la composition dentifrice.

Ladite composition dentifrice peut comprendre en outre d'autres ingrédients habituels, en particulier des agents abrasifs minéraux, insolubles dans l'eau, éventuellement des agents épaississants autres, des humectants ...

Comme agents abrasifs, on peut mentionner en particulier les silices abrasives, le carbonate de calcium, l'alumine hydratée, la bentonite, le silicate d'aluminium, le silicate de zirconium, les métaphosphates et phosphates de sodium, de potassium, de calcium et de magnésium. La quantité totale de poudre(s) abrasive(s) peut constituer de l'ordre de 5 à 50 % du poids de la composition dentaire.

Parmi les agents épaississants autres, on peut mentionner, la gomme xanthane, la gomme guar, les carraghénanes, les dérivés de la cellulose, les alginates, en quantité pouvant aller jusqu'à 5 % du poids de ladite composition ...

Parmi les agents humectants on peut citer par exemple le glycérol, le sorbitol, les polyéthylène glycols, les polypropylène glycols, le xylitol, en quantité de l'ordre de 2 à 85 %, de préférence de l'ordre de 3 à 55 % du poids de composition dentifrice exprimé en sec.

Ces compositions peuvent en outre comporter notamment des agents tensio-actifs, des agents détergents, des colorants, des antibactériens, des dérivés fluorés, des opacifiants, des arômes, des édulcorants, des agents antitartre, antiplaque, des agents de blanchiment, du bicarbonate de sodium, des antiseptiques, des enzymes, des extraits naturels (camomille, thym...).

Les exemples suivants illustrent l'invention sans toutefois en limiter la portée.

EXEMPLES 1-3

Pâte dentifrice opaque modèle

- sorbitol (Neosorb 70/70 (société ROQUETTE FRERES)) 45
- polyethylene glycol PEG 1500 5
- saccharinate de sodium 0,2
- fluorure de sodium 0,08

- monofluorophosphate de sodium 0,72
- eau 24,2
- silice abrasive (Tixosil 63 commercialisé par la société RHODIA) 10
- silice de l'invention 7
- dioxyde de titane 1
- arôme spearmint 1
- agent moussant (30 % dans eau) : Texapon Z 95 P (société COGNIS) 5

Mesure de la viscosité d'une formulation dentifrice

La viscosité est déterminée sur tube de pâte de 25 mm de diamètre, à des périodes déterminées à 37 °C après préparation de la pâte.

Le matériel de mesure utilisé est un viscosimètre Brookfield RVT équipé d'un dispositif hélipath. La pige T - E est utilisée à 5 r.p.m (tours par minute). La mesure est effectuée en descente après 90 secondes.

EXEMPLE 1

Dans un réacteur équipé d'un système de régulation de température et de pH et d'un système d'agitation avec hélice 3 pâles, on a introduit 14000 g d'eau, et 450 g d'une solution aqueuse de silicate de sodium à 236 g/L en équivalent $SiO_2$, le rapport pondéral (Rp) $SiO_2/Na_2O$ du silicate de sodium utilisé étant de 3,46.

Après la mise en marche de l'agitation (250 tours par minute) le pied de cuve ainsi constitué a été chauffé à 95 °C et le pH a été amené à 7,5, en 11 minutes, par ajout d'une solution aqueuse d'acide sulfurique à 80 g/L (débit moyen de 61 g par minute).

Une fois le pH de 7,5 atteint, on a procédé à une addition continue de 3045 g d'une solution aqueuse de silicate de sodium (Rp = 3,46) à 236 g/L en équivalent $SiO_2$, à un débit constant de 35 grammes par minute (durée de l'addition : 87 minutes), en maintenant le pH du milieu à une valeur égale à 7,5 (à 0,1 unité de pH près), par addition dans le milieu d'une solution aqueuse d'acide sulfurique à 80 g/L avec un débit contrôlé en fonction de l'évolution mesurée pour le pH du milieu. En bilan, 3383 g de la solution d'acide sulfurique ont été ajoutés dans le milieu, ce qui correspond à un débit moyen de 40 grammes de solution d'acide sulfurique ajoutés par minute.

Après les 87 minutes d'addition, on a arrêté l'addition de silicate et on a poursuivi une addition d'acide jusqu'à stabilisation du pH du mélange réactionnel à 3,6. On a effectué un mûrissement en laissant la solution sous agitation pendant 5 minutes.

La bouillie obtenue a ensuite été filtrée et lavée sur filtre plan, puis le gâteau obtenu, dont la perte au feu est de 80,5 %, a été délité mécaniquement à un pH de 5,5 , puis a été séché par atomisation à turbine.

Les caractéristiques physico-chimiques de la silice sèche non broyée obtenue sont les suivantes :

- pH : 5,9
- diamètre médian de particules : 80 $\mu$m
- diamètre médian après ultra-sons : 31,0 $\mu$m
- % supérieur à 51 $\mu$m après ultra-sons : 18,6
- teneur en $NaSO_4$ : 1,6 % en masse (par rapport à la masse totale du matériau à l'état sec)
- surface spécifique CTAB : 133 m$^2$/g
- surface spécifique BET : 143 m$^2$/g
- prise d'huile DOP : 305 ml/100g
- perte au feu à 1000 °C : 6,5 %
- teneur résiduelle en eau après 2 heure à 105 °C : 3,9 %
- reprise en eau : 5,8 %
- transmission : 80 % à un indice de réfraction de 1,460
- densité de remplissage à l'état tassé (DRT) : 0,27 g/ml
- viscosité de la pâte dentifrice modèle après 4 semaines : 250 mPa.s

EXEMPLE 2

On répète les opérations décrites à l'exemple 1 comparatif, en broyant le produit séché, de manière à obtenir un diamètre médian de particules de 10 $\mu$m.

Les caractéristiques physico-chimiques de la silice sèche broyée obtenue sont les suivantes :

- pH : 5,9

- diamètre médian de particules : 10 $\mu$m
- diamètre médian après ultra-sons : 7 $\mu$m
- % supérieur à 51 $\mu$m après ultra-sons : 1,0
- teneur en $NaSO_4$ : 1,6 % en masse (par rapport à la masse totale du matériau à l'état sec)
- surface spécifique CTAB : 133 $m^2$/g
- surface spécifique BET : 143 $m^2$/g
- prise d'huile DOP : 315 ml/100g
- perte au feu à 1000 °C : 7 %
- teneur résiduelle en eau après 2 heure à 105° C : 4,4 %
- reprise en eau : 5,9 %
- transmission : 80 % à un indice de réfraction de 1,460
- densité de remplissage à l'état tassé (DRT) : 0,1 g/ml
- viscosité de la pâte dentifrice modèle après 4 semaines : 325 mPa.s

## EXEMPLE 3

Dans un réacteur équipé d'un système de régulation de température et de pH et d'un système d'agitation avec hélice 3 pâles, on a introduit 14000 g d'eau, et 630 g d'une solution aqueuse de silicate de sodium à 236 g/L en équivalent $SiO_2$, le rapport pondéral (Rp) $SiO_2$/$Na_2O$ du silicate de sodium utilisé étant de 3,46.

Après la mise en marche de l'agitation (250 tours par minute) le pied de cuve ainsi constitué a été chauffé à 95 °C et le pH a été amené à 7,5, en 11 minutes, par ajout d'une solution aqueuse d'acide sulfurique à 80 g/L (débit moyen de 61 g par minute).

Une fois le pH de 7,5 atteint, on a procédé à une addition continue de 3600 g d'une solution aqueuse de silicate de sodium (Rp = 3,46) à 236 g/L en équivalent $SiO_2$, à un débit constant de 48 grammes par minute (durée de l'addition : 75 minutes), en maintenant le pH du milieu à une valeur égale à 7,5 (à 0,1 unité de pH près), par addition dans le milieu d'une solution aqueuse d'acide sulfurique à 80 g/L avec un débit contrôlé en fonction de l'évolution mesurée pour le pH du milieu. En bilan, 3975 g de la solution d'acide sulfurique ont été ajoutés dans le milieu, ce qui correspond à un débit moyen de 53 grammes de solution d'acide sulfurique ajoutés par minute.

Après les 90 minutes d'addition, on a arrêté l'addition de silicate et on a poursuivi une addition d'acide jusqu'à stabilisation du pH du mélange réactionnel à 3,4. On a effectué un mûrissement en laissant la solution sous agitation pendant 5 minutes.

La bouillie obtenue a ensuite été filtrée et lavée sur filtre plan, puis le gâteau obtenu, dont la perte au feu est de 86 %, a été délité mécaniquement à un pH de 5, puis a été séché par atomisation à turbine.

Les caractéristiques physico-chimiques de la silice sèche non-broyée obtenue sont les suivantes :

- pH : 5,3
- diamètre médian de particules :65 $\mu$m
- diamètre médian après ultra-sons : 22 $\mu$m
- % supérieur à 51 $\mu$m après ultra-sons : 3,3
- teneur en $NaSO_4$ : 1,0 % en masse (par rapport à la masse totale du matériau à l'état sec)
- surface spécifique CTAB : 182 $m^2$/g
- surface spécifique BET : 185 $m^2$/g
- prise d'huile DOP : 340 ml/100g
- perte au feu à 1000 °C : 6,5 %
- teneur résiduelle en eau après 2 heure à 105 °C :3,9 %
- reprise en eau : 5,7 %
- transmission : 85 % à un indice de réfraction de 1,460
- densité de remplissage à l'état tassé (DRT) : 0,18 g/ml
- viscosité de la pâte dentifrice modèle après 4 semaines : 615 mPa.s

## EXEMPLE 4

Dans un réacteur équipé d'un système de régulation de température et de pH et d'un système d'agitation avec hélice 3 pâles, on a introduit 14000 g d'eau, et 450 g d'une solution aqueuse de silicate de sodium à 236 g/L en équivalent $SiO_2$, le rapport pondéral (Rp) $SiO_2$/$Na_2O$ du silicate de sodium utilisé étant de 3,46.

Après la mise en marche de l'agitation (250 tours par minute) le pied de cuve ainsi constitué a été chauffé à 98 °C et le pH a été amené à 7,5, en 11 minutes, par ajout d'une solution aqueuse d'acide sulfurique à 80 g/L (débit moyen de 61 g par minute).

Une fois le pH de 7,5 atteint, on a procédé à une addition continue de 3150 g d'une solution aqueuse de silicate de sodium (Rp = 3,46) à 236 g/L en équivalent SiO$_2$, à un débit constant de 35 grammes par minute (durée de l'addition : 90 minutes), en maintenant le pH du milieu à une valeur égale à 7,5 (à 0,1 unité de pH près), par addition dans le milieu d'une solution aqueuse d'acide sulfurique à 80 g/L avec un débit contrôlé en fonction de l'évolution mesurée pour le pH du milieu. En bilan, 3510 g de la solution d'acide sulfurique ont été ajoutés dans le milieu, ce qui correspond à un débit moyen de 39 grammes de solution d'acide sulfurique ajoutés par minute.

Après les 90 minutes d'addition, on a arrêté l'addition de silicate et on a poursuivi une addition d'acide jusqu'à stabilisation du pH du mélange réactionnel à 3,4. On a effectué un mûrissement en laissant la solution sous agitation pendant 5 minutes.

La bouillie obtenue a ensuite été filtrée et lavée sur filtre plan, puis le gâteau obtenu, dont la perte au feu est de 86,4%, a été délité mécaniquement à un pH de 4,3, puis a été séché par atomisation à turbine.

La silice séchée a ensuite été broyée à l'aide d'un broyeur sélecteur à marteaux.

Les caractéristiques physico-chimiques de la silice sous forme de poudre obtenue sont les suivantes :

- pH : 4,6
- taille moyenne de particules : 12 $\mu$m
- teneur en NaSO$_4$ : 0,25 % en masse (par rapport à la masse totale du matériau à l'état sec)
- surface spécifique CTAB : 166 m$^2$/g
- surface spécifique BET : 170 m$^2$/g
- prise d'huile DOP : 365 ml/100g
- perte au feu à 1000 °C : 5 %
- teneur résiduelle en eau après 2 heures à 105 °C : 2,5 %
- reprise en eau : 5,8 %
- densité de remplissage à l'état tassé (DRT) : 0,08 g/ml

**Revendications**

1. Silice de précipitation présentant :

    • une surface spécifique CTAB de 140 à 230 m$^2$/g, de préférence de 145 à 195 m$^2$/g, tout particulièrement .de 145 à 185 m$^2$/g, tout particulièrement de 150 à 185 m$^2$/g, notamment de 150 à 180 m$^2$/g
    • une prise d'huile DOP supérieure à 300 ml/100g, de préférence supérieure à 310 ml/100g, plus préférentiellement de 315 à 450 ml/100g, tout particulièrement de 320 à 400 ml/100g, notamment de 340 à 380 ml/100g
    • une reprise en eau inférieure à 6 % en masse et de préférence supérieure à 3 % en masse, tout particulièrement supérieure ou égale à 4 % en masse et inférieure ou égale à 5,8 % en masse
    • un pH de 3,5 à 7,5, de préférence de 4 à 7, tout particulièrement de 4 à 6
    • un taux d'anion résiduel, exprimé en sulfate de sodium, inférieur ou égal à 2 % en masse, de préférence inférieur ou égal à 1,5 % en masse, particulièrement inférieur ou égal à 1 % en masse et notamment inférieur ou égal à 0,5 % en masse
    • une taille moyenne ou un diamètre médian de particules inférieur à 30 $\mu$m ou compris entre 30 $\mu$m et 20 mm.

2. Silice selon la revendication 1, **caractérisée en ce qu'**elle présente une taille moyenne ou un diamètre médian de particules inférieur à 30 $\mu$m, de préférence inférieur à 20 $\mu$m, en particulier compris entre 5 et 15 $\mu$m, notamment entre 8 et 13 $\mu$m.

3. Silice selon la revendication 1, **caractérisée en ce qu'**elle présente une taille moyenne ou un diamètre médian de particules compris entre 30 $\mu$m et 20 mm.

4. Silice selon l'une des revendications 1 à 3, **caractérisée en ce qu'**elle présente un diamètre médian des particules, après désagglomération aux ultra-sons, d'au plus 35 $\mu$m, de préférence d'au plus 30 $\mu$m, tout particulièrement d'au plus 25 $\mu$m.

5. Silice selon l'une des revendications 1. à 4, **caractérisée en ce qu'**elle présente une surface spécifique BET telle que la différence BET-CTAB soit d'au plus 30m$^2$/g, de préférence d'au plus 25 m$^2$/g, plus préférentiellement d'au plus 20 m$^2$/g, en particulier d'au plus 10 m$^2$/g.

6. Silice selon l'une des revendications 1 à 5, **caractérisée en ce qu'**elle présente une densité de remplissage à l'état tassé d'au plus 0,3 g/ml, de préférence de 0,04 à 0,3 g/ml.

7. Silice selon l'une des revendications 1 à 6, **caractérisée en ce qu'**elle se présente sous forme de poudre.

8. Procédé de préparation d'une silice selon l'une des revendications 1 à 7, comprenant les étapes suivantes :

• (a) réaliser un pied de cuve initial de température comprise entre 80 et 100 °C, de préférence supérieure ou égale à 90 °C, comprenant de l'eau et un silicate, la concentration en silicate dans ledit pied de cuve, exprimée en équivalent $SiO_2$, étant inférieure ou égale à 15 g/L ;
• (b) addition, à une température comprise entre 80 et 100 °C, de préférence 90 et 100 °C, d'un agent acidifiant, amener le pH du milieu jusqu'à une valeur comprise entre 7 et 8, de préférence jusqu'à une valeur comprise entre 7,2 et 7,8, et avantageusement entre 7,3 et 7,7 (typiquement jusqu'à une valeur sensiblement égale à 7,5) ;
• (c) dans le milieu ainsi réalisé, effectuer, à une température comprise entre 80 et 100 °C, de préférence entre 90 et 100 °C, l'addition simultanée d'un silicate et d'un agent acidifiant, les quantités respectives de silicate et d'agent acidifiant ajoutées au cours du temps étant spécifiquement choisies de façon à ce que, pendant toute la durée de l'addition :

- le pH du milieu réactionnel reste compris entre 7 et 8, et avantageusement entre 7,2 et 7,8 ; et
- la concentration en silicium dans le milieu, exprimée en équivalent $SiO_2$, reste inférieure ou égale à 35 g/L ;

• (d) ajouter, à une température comprise entre 80 et 100 °C, de préférence entre 90 et 100 °C, un agent acidifiant au milieu obtenu à l'issue de l'étape (c) de façon à porter le milieu à un pH compris entre 3 et 6,5 ;
• (e) filtrer la dispersion aqueuse de silice obtenue ;
• (f) sécher le gâteau de filtration réalisé à l'issu de la filtration, de préférence en le lavant, au préalable ;
• (g) éventuellement, broyer ou microniser la silice obtenue à l'issue de l'étape (f),

ledit procédé étant **caractérisé en ce que** le gâteau de filtration présente, préalablement à son séchage à l'étape (f), une perte au feu à 1000 °C supérieure à 82 % en masse, de préférence d'au moins 84 % en masse, tout particulièrement de 84 à 88 % en masse.

9. Utilisation d'une silice selon l'une des revendications 1 à 7 ou obtenue par le procédé selon la revendication 8, à titre de charge renforçante dans une matrice à base d'élastomère(s), en particulier clair(s) ou semi-clair(s), pour semelles de chaussures.

10. Utilisation d'une silice selon l'une des revendications 1 à 7 ou obtenue par le procédé selon la revendication 8, à titre de charge renforçante dans une matrice à base de silicone(s).

11. Utilisation d'une silice selon l'une des revendications 1 à 7 ou obtenue par le procédé selon la revendication 8 à titre de support de liquide.

12. Utilisation d'une silice selon l'une des revendications 1 à 7 ou obtenue par le procédé selon la revendication 8, à titre de charge épaississante, de support et/ou d'excipients dans une matrice organique ou aqueuse pâteuse, gel, solide ou liquide.

13. Utilisation selon la revendication 12 à titre de charge épaississante, dans une composition dentifrice sous forme de pâte ou de gel.

14. Utilisation d'une silice selon l'une des revendications 1 à 7 ou obtenue par le procédé selon la revendication 8 pour la préparation de séparateurs de batterie.

**Patentansprüche**

1. Fällungskieselsäure, welche aufweist:

- eine spezifische Oberfläche CTAB von 140 bis 230 m$^2$/g, vorzugsweise von 145 bis 195 m$^2$/g, insbesondere von 145 bis 185 m$^2$/g, insbesondere von 150 bis 185 m$^2$/g, bevorzugt von 150 bis 180 m$^2$/g,
- eine Ölaufnahme DOP von mehr als 300 ml/100 g, vorzugsweise mehr als 310 ml/100 g, bevorzugter von 315 bis 450 ml/100 g, insbesondere von 320 bis 400 ml/100 g, bevorzugt von 340 bis 380 ml/100 g,
- eine Wasseraufhahme von weniger als 6 Masse-% und vorzugsweise mehr als 3 Masse-%, insbesondere größer oder gleich 4 Masse-% und kleiner oder gleich 5,8 Masse-%,
- einen pH von 3,5 bis 7,5, vorzugsweise von 4 bis 7, insbesondere von 4 bis 6,
- einen Anionenrestgehalt, ausgedrückt als Natriumsulfat, kleiner oder gleich 2 Masse-%, vorzugsweise kleiner oder gleich 1,5 Masse-%, insbesondere kleiner oder gleich 1 Masse-% und bevorzugt kleiner oder gleich 0,5 Masse-%,
- eine mittlere Partikelgröße oder einen mittleren Partikeldurchmesser von weniger als 30 $\mu$m oder zwischen 30 $\mu$m und 20 mm.

2. Kieselsäure nach Anspruch 1,
**dadurch gekennzeichnet, dass** diese eine mittlere Partikelgröße oder einen mittleren Partikeldurchmesser von weniger als 30 $\mu$m, vorzugsweise weniger als 20 $\mu$m, insbesondere zwischen 5 und 15 $\mu$m, bevorzugt zwischen 8 und 13 $\mu$m, aufweist.

3. Kieselsäure nach Anspruch 1, **dadurch gekennzeichnet, dass** diese eine mittlere Partikelgröße oder einen mittleren Partikeldurchmesser zwischen 30 $\mu$m und 20 mm aufweist.

4. Kieselsäure nach einem der Ansprüche 1 bis 3, **dadurch gekennzeichnet, dass** diese einen mittleren Partikeldurchmesser, nach Ultraschall-Desagglomeration, von höchstens 35 $\mu$m, vorzugsweise von höchstens 30 $\mu$m, insbesondere von höchstens 25 $\mu$m aufweist.

5. Kieselsäure nach einem der Ansprüche 1 bis 4, **dadurch gekennzeichnet, dass** diese eine spezifische Oberfläche BET derart aufweist, dass die Differenz BET-CTAB höchstens 30 m$^2$/g, vorzugsweise höchstens 25 m$^2$/g, bevorzugter höchstens 20 m$^2$/g und insbesondere höchstens 10 m$^2$/g, beträgt.

6. Kieselsäure nach einem der Ansprüche 1 bis 5, **dadurch gekennzeichnet, dass** diese eine Füllungsdichte im kompaktierten Zustand von höchstens 0,3 g/ml, vorzugsweise von 0,04 bis 0,3 g/ml, aufweist.

7. Kieselsäure nach einem der Ansprüche 1 bis 6, **dadurch gekennzeichnet, dass** diese in der Form eines Pulvers vorliegt.

8. Verfahren zur Herstellung einer Kieselsäure nach einem der Ansprüche 1 bis 7, umfassend die folgenden Schritte :

(a) Herstellen eines anfänglichen Starters bei einer Temperatur zwischen 80 und 100°C, vorzugsweise größer oder gleich 90°C, umfassend Wasser und ein Silikat, wobei die Silikat-Konzentration in dem Starter, ausgedrückt als SiO$_2$-Äquivalent, kleiner oder gleich 15 g/l ist;
(b) Zusetzen, bei einer Temperatur zwischen 80 und 100°C, vorzugsweise 90 und 100°C, eines Ansäuerungsmittels, Führen des pH des Mediums auf einen Wert zwischen 7 und 8, vorzugsweise auf einen Wert zwischen 7,2 und 7,8 und vorteilhaft zwischen 7,3 und 7,7 (typischerweise auf einen Wert im Wesentlichen gleich 7,5);
(c) in dem so hergestellten Medium, Durchführen, bei einer Temperatur zwischen 80 und 100°C, vorzugsweise zwischen 90 und 100°C, eines gleichzeitigen Zusetzens eines Silikats und eines Ansäuerungsmittels, wobei die jeweiligen Mengen an Silikat und Ansäuerungsmittel, die im Lauf der Zeit zugesetzt werden, spezifisch derart ausgewählt werden, dass während der gesamten Dauer des Zusetzens :

- der pH des Reaktionsmediums zwischen 7 und 8, und vorteilhaft zwischen 7,2 und 7,8, bleibt; und
- die Silicium-Konzentration in dem Medium, ausgedrückt als SiO$_2$-Äquivalent, kleiner oder gleich 35 g/l bleibt;

(d) Zusetzen, bei einer Temperatur zwischen 80 und 100°C, vorzugsweise zwischen 90 und 100°C, eines Ansäuerungsmittels zu dem Medium, das aus Schritt (c) erhalten wird, um das Medium auf einen pH zwischen 3, und 6,5 zu führen;
(e) Filtrieren der erhaltenen wässrigen Kieselsäure-Dispersion;
(f) Trocknen des Filterkuchens, der aus der Filtration erhalten wird, wobei dieser vorzugsweise zuvor gewaschen wird;
(g) gegebenenfalls Zerkleinern oder Mikronisieren der Kieselsäure, die aus Schritt (f) erhalten wird;

wobei das Verfahren **dadurch gekennzeichnet ist, dass** der Filterkuchen, vor seiner Trocknung in Schritt (f), einen Glühverlust bei 1000°C von mehr als 82 Masse-%, vorzugsweise von mindestens 84 Masse-%, insbesondere von 84 bis 88 Masse-%, aufweist.

9. Verwendung einer Kieselsäure nach einem der Ansprüche 1 bis 7, oder erhalten durch das Verfahren nach Anspruch 8, als Verstärkungscharge in einer Matrix auf der Basis von Elastomer(en), insbesondere transparent oder halbtransparent, für Schuhsohlen.

10. Verwendung einer Kieselsäure nach einem der Ansprüche 1 bis 7, oder erhalten durch das Verfahren nach Anspruch 8, als Verstärkungscharge in einer Matrix auf der Basis von Silikon(en).

11. Verwendung einer Kieselsäure nach einem der Ansprüche 1 bis 7, oder erhalten durch das Verfahren nach Anspruch 8, als Flüssigkeitsträger.

12. Verwendung einer Kieselsäure nach einem der Ansprüche 1 bis 7, oder erhalten durch das Verfahren nach Anspruch 8, als Verdickungscharge, als Träger und/oder Exzipienten in einer organischen oder wässrigen pastenartigen, gelartigen, festen oder flüssigen Matrix.

13. Verwendung nach Anspruch 12, als Verdickungscharge in einer Zahnpastazusammensetzung in der Form einer Paste oder eines Gels.

14. Verwendung einer Kieselsäure nach einem der Ansprüche 1 bis 7, oder erhalten durch das Verfahren nach Anspruch 8, zur Herstellung von Batterieseparatoren.

## Claims

1. Precipitated silica exhibiting :

   - a CTAB specific surface of 140 to 230 $m^2/g$, preferably of 145 to 195 $m^2/g$, more preferably of 145 to 185 $m^2/g$, very particularly of 150 to 185 $m^2/g$, in particular of 150 to 180 $m^2/g$,
   - a DOP oil uptake of greater than 300 ml/100 g, preferably of greater than 310 ml/100 g, more preferably of 315 to 450 ml/100 g, very particularly of 320 to 400 ml/100 g, in particular of 340 to 380 ml/100 g,
   - a water uptake of less than 6% by weight and preferably of greater than 3% by weight, very particularly of greater than or equal to 4% by weight and of less than or equal to 5.8% by weight,
   - a pH of 3.5 to 7.5, preferably of 4 to 7, very particularly of 4 to 6,
   - a level of residual anion, expressed as sodium sulfate, of less than or equal to 2% by weight, preferably of less than or equal to 1.5% by weight, particularly of less than or equal to 1% by weight and in particular of less than or equal to 0.5% by weight,
   - a mean particle size or a median particle diameter of less than 30 $\mu$m or of between 30 $\mu$m and 20 mm.

2. Silica according to Claim 1, **characterized in that** it exhibits a mean particle size or a median particle diameter of less than 30 $\mu$m, preferably of less than 20 $\mu$m, in particular of between 5 and 15 $\mu$m, especially between 8 and 13 $\mu$m.

3. Silica according to Claim 1, **characterized in that** it exhibits a mean particle size or a median particle diameter of between 30 $\mu$m and 20 mm.

4. Silica according to one of Claims 1 to 3, **characterized in that** it exhibits a median particle diameter, after deagglomeration under ultrasound, of at most 35 $\mu$m, preferably of at most 30 $\mu$m, very particularly of at most 25 $\mu$m.

5. Silica according to one of Claims 1 to 4, **characterized in that** it exhibits a BET specific surface such that the BET-CTAB difference is at most 30 $m^2/g$, preferably at most 25 $m^2/g$, more preferably at most 20 $m^2/g$, in particular at most 10 $m^2/g$.

6. Silica according to one of Claims 1 to 5, **characterized in that** it exhibits a packing density of at most 0.3 g/ml, preferably of 0.04 to 0.3 g/ml.

7. Silica according to one of Claims 1 to 6, **characterized in that** it is provided in the form of a powder.

8. Process for the preparation of a silica according to one of Claims 1 to 7, comprising the following stages :

(a) producing a starting vessel heel with a temperature of between 80 and 100°C, preferably of greater than or equal to 90°C, comprising water and a silicate, the concentration of silicate in said vessel heel, expressed as $SiO_2$ equivalent, being less than or equal to 15 g/l;
(b) adding, at a temperature of between 80 and 100°C, preferably 90 and 100°C, an acidifying agent to bring the pH of the medium to a value of between 7 and 8, preferably to a value of between 7.2 and 7.8 and advantageously between 7.3 and 7.7 (typically to a value substantially equal to 7.5);
(c) in the medium thus produced, carrying out, at a temperature of between 80 and 100°C, preferably between 90 and 100°C, the simultaneous addition of a silicate and of an acidifying agent, the respective amounts of silicate and of acidifying agent added over time being specifically chosen so that, throughout the duration of the addition :

- the pH of the reaction medium remains between 7 and 8 and advantageously between 7.2 and 7.8; and
- the concentration of silicon in the medium, expressed as $SiO_2$ equivalent, remains less than or equal to 35 g/l;

(d) adding, at a temperature of between 80 and 100°C, preferably between 90 and 100°C, an acidifying agent to the medium obtained on conclusion of stage (c) so as to bring the medium to a pH of between 3 and 6.5;
(e) filtering the aqueous silica dispersion obtained;
(f) drying the filtration cake produced on conclusion of the filtration, preferably washing it beforehand;
(g) optionally milling or micronizing the silica obtained on conclusion of stage (f),

said process being **characterized in that** the filtration cake exhibits, prior to the drying of it in stage (f), a loss on ignition at 1000°C of greater than 82% by weight, preferably of at least 84% by weight, very particularly of 84 to 88% by weight.

9. Use of a silica according to one of Claims 1 to 7 or obtained by the process according to Claim 8 as reinforcing filler in a matrix based on elastomer(s), in particular clear or semi-clear elastomer(s), for shoe soles.

10. Use of a silica according to one of Claims 1 to 7 or obtained by the process according to Claim 8 as reinforcing filler in a matrix based on silicone(s).

11. Use of a silica according to one of Claims 1 to 7 or obtained by the process according to Claim 8 as carrier for liquids.

12. Use of a silica according to one of Claims 1 to 7 or obtained by the process according to Claim 8 as thickening filler, carrier and/or excipients in a pasty, gel, solid or liquid organic or aqueous matrix.

13. Use according to Claim 12 as thickening filler in a dentifrice composition in the paste or gel form.

14. Use of a silica according to one of Claims 1 to 7 or obtained by the process according to Claim 8 for the preparation of battery separators.

**EP 1 694 601 B1**

## RÉFÉRENCES CITÉES DANS LA DESCRIPTION

**Documents brevets cités dans la description**

- WO 03055801 A

- WO 0193803 A

**Littérature non-brevet citée dans la description**

- **BRUNAUER - EMET - TELLER.** *The journal of the American Chemical Society,* Février 1938, vol. 60, 309